# FASCICULE DE BREVET EUROPEEN

(11) **EP 1 819 369 B1**
(45) Date de publication et mention de la délivrance du brevet: **17.02.2010**
(21) Numéro de dépôt: 05825978.9
(22) Date de dépôt: 07.12.2005
(51) Int. Cl.: A61L 9/18, A61L 9/20, F24F 3/16

(54) **INACTIVATION D'AGENTS BIOLOGIQUES DISPERSES EN MILIEU GAZEUX PAR UN SEMI-CONDUCTEUR PHOTOACTIF**
INAKTIVIERUNG VON BIOLOGISCHEN MITTELN, DIE IN EINEM GASFÖRMIGEN MEDIUM DISPERGIERT SIND, MIT EINEM LICHTAKTIVIERTEN HALBLEITER
INACTIVATING BIOLOGICAL AGENTS DISPERSED IN GASEOUS MEDIUM WITH A PHOTOACTIVATED SEMICONDUCTOR

(30) Priorité: 09.12.2004 FR 0413152
(43) Date de publication de la demande: 22.08.2007
(73) Titulaire: CENTRE NATIONAL DE LA RECHERCHE SCIENTIFIQUE (CNRS), 75016 Paris (FR); UNIVERSITE LOUIS PASTEUR STRASBOURG 1, 67037 Strasbourg Cedex (FR)
(72) Inventeur: GARIN, François, F-67300 Schiltigheim (FR); KELLER, Nicolas, F-67200 Strasbourg (FR); LETT, Marie-claire, F-67300 Schiltigheim (FR); KELLER-SPITZER, Valérie, 67203 Oberschaeffolsheim (FR); LEDOUX, Marc, F-67000 Strasbourg (FR)
(74) Mandataire: Colombet, Alain André
(86) Numéro de dépôt international: PCT/FR2005/003074
(87) Numéro de publication internationale: WO 2006/061518

(56) Documents cités:
- EP-A- 0 978 690
- WO-A-97/09073
- DE-A1- 19 906 113
- GB-A- 2 367 495

## Description

La présente invention a trait à la décontamination de milieux gazeux comprenant des espèces biologiques, et notamment le traitement d'air contaminé par des bactéries ou des virus.

On rencontre dans de nombreux domaines des flux gazeux véhiculant, en suspension, des espèces biologiques qui peuvent s'avérer nocives ou toxiques à plus ou moins long terme. A titre d'exemple, on peut en particulier citer l'air des systèmes de climatisation ou bien l'air issu de tours aéroréfrigérées, qui contiennent de fines gouttelettes d'eau en suspension (aérosols) qui peuvent renfermer des bactéries nocives telles que celles du genre *Legionella,* comme *Legionella pneumophilia* responsable de la légionellose. Un autre exemple de flux gazeux contaminé est celui de l'air circulant au sein des hôpitaux, qui est susceptible de véhiculer des virus ou des bactéries pouvant induire des infections nosocomiales. De façon plus générale, l'air présent dans tout milieu confiné ou à forte densité de population est susceptible de véhiculer, en quantité plus ou moins importante, des espèces biologiques de type bactéries, virus ou spores, qu'il peut être souhaitable d'éliminer.

A l'heure actuelle, pour réaliser la décontamination de tels milieux gazeux viciés, la plupart des solutions proposées sont relativement coûteuses et/ou complexes à mettre en oeuvre, et leur l'efficacité n'est en outre pas toujours satisfaisante.

Dans ce cadre, il a été proposé des systèmes de filtration des flux gazeux. De tels systèmes de filtration impliquent en général un coût important, notamment dans la mesure où les espèces biologiques qu'on cherche à piéger sont généralement de taille très faible. De plus, ces systèmes de filtration présentent l'inconvénient de devoir être remplacés relativement souvent.

En marge des systèmes de filtration, il a également été proposé d'autres procédés de traitement de milieux gazeux, tels que par exemple des traitements thermiques ou des traitements par des désinfectants chimiques. Ces procédés sont toutefois contraignants, dans la mesure où ils impliquent en général une indisponibilité des zones touchées pendant le traitement. De plus, certaines espèces biologiques (notamment certaines souches de *Legionalla pneumophilia*) se révèlent résistantes à ce type de traitement thermique ou chimique.

Plus récemment, il a été décrit, notamment dans les demandes de brevets DE-A-19906113, GB-A-2367495, WO 97/09073 et EP 978 690, des procédés de destruction de microorganismes au contact de photocatalyseurs activés sous UV.

Un but de la présente invention est de fournir un procédé de traitement des flux gazeux précités, qui soit peu onéreux, simple à mettre en pratique, et au moins aussi efficace, et de préférence plus efficace, que les procédés de décontamination actuellement connus.

A cet effet, selon un premier aspect, la présente invention a pour objet un procédé d'inactivation d'agents biologiques dispersés au sein d'un milieu gazeux, qui comprend une étape consistant à mettre ledit milieu gazeux en contact avec un matériau semi-conducteur photoactivé au sein d'un réacteur dont la surface interne comporte une pluralité de saillies disposées en hélice autour d'une ligne, ledit matériau semi-conducteur photoactivé étant mis en oeuvre sous la forme d'un dépôt sur la surface interne dudit réacteur, au moins sur lesdites saillies. De préférence, le matériau semi-conducteur photoactivé utilisé à cet effet est de l'oxyde de titane photoactivé.

Selon un mode de réalisation particulièrement intéressant, le procédé de l'invention est conduit dans un dispositif qui comporte :
(i) ledit réacteur, ce réacteur comprenant :
   - une surface interne délimitant un passage s'étendant suivant une ligne, ladite surface interne comportant une pluralité de saillies disposées en hélice autour de cette ligne,
   - une entrée adaptée à l'introduction du milieu gazeux contenant les agents biologiques dispersés dans le passage, et
   - une sortie adaptée à l'évacuation du milieu après traitement, ledit réacteur comprenant ledit dépôt de matériau semi-conducteur déposé sur les saillies de sa surface interne, et
(ii) des moyens d'irradiation dudit dépôt de matériau semi-conducteur, qui photoactivent le matériau semi-conducteur du dépôt en présence du milieu gazeux contenant les agents biologiques.

Les inventeurs ont maintenant mis en évidence que le dépôt d'un matériau semi-conducteur du type TiO₂ photoactivé sur des saillies de la surface interne d'un réacteur disposées sous forme d'hélice permet une inactivation particulièrement efficace des agents biologiques.

La présence de saillies sur la surface interne du réacteur présente, entre autres, l'avantage d'accroître la surface interne du réacteur, ce qui permet d'accroître la surface d'échange entre le matériau semi-conducteur de type oxyde de titane et les agents biologiques du milieu gazeux.

De plus, ces saillies autorisent le dépôt d'une quantité plus importante de matériau semi-conducteur de type oxyde de titane sur la surface interne du réacteur. Ainsi, par exemple, dans le cas où le dépôt de matériau semi-conducteur est effectué par dépôt d'une dispersion de particules dudit matériau sur la surface interne du réacteur, puis séchage (ce qui est souvent le cas en pratique), la présence de saillies crée une "rugosité" de surface qui permet d'augmenter la quantité de matériau semi-conducteur de type oxyde de titane qui peut être déposée sur la surface interne du réacteur.

En outre, la présence des saillies précitées permet une mise en contact de la totalité du flux gazeux avec l'oxyde de titane présent sur la surface interne du réacteur, notamment en induisant un régime turbulent (ou tout au moins non laminaire) au sein du réacteur, ce qui augmente encore les probabilités de rencontre entre les agents biologiques et l'oxyde de titane photoactivé.

Par ailleurs, la disposition spécifique des saillies en hélice permet de limiter les pertes de charge au sein du réacteur, tout en maintenant des probabilités élevées de contact entre les agents biologiques du flux gazeux et l'oxyde de titane photoactivé. En fait, les inventeurs ont maintenant mis en évidence que cette disposition des saillies sur la surface interne du réacteur en hélice autour d'une ligne conduit à un compromis idéal entre perte de charge réduite et probabilité élevée de contact entre les agents biologiques et le matériau semi-conducteur photoactivé.

Par "agents biologiques", on entend, au sens de la présente description, des entités de nature biologique, généralement de faible taille, typiquement entre 0,05 µm (microns) et 10 µm (microns), et susceptibles de pouvoir être véhiculées par un courant gazeux. Ainsi, les agents biologiques à inactiver selon le procédé de l'invention peuvent notamment être des bactéries (bactéries du genre *Legionella,* comme *Legionella pneumophilia* par exemple), des virus, des spores, des champignons, ou bien encore un mélange de telles entités.

Le terme "agent biologique inactivé" désigne quant à lui, au sens de la présente description, un agent du type précité ayant perdu une activité biologique, et notamment ayant perdu sa capacité de réplication (ou de reproduction).

En particulier, on entend par "bactérie inactivée", au sens de la présente description, une bactérie incapable de développer une colonie après mise en culture dans un milieu adapté. Ainsi, sont notamment considérées comme des "bactéries inactivées" :
- des bactéries "mortes" (typiquement des bactéries au sein desquelles on ne détecte pas de phénomènes de respiration) ; et
- des bactéries qui, bien que vivantes, ne se développent pas après mise en culture.

En général, le procédé de l'invention est conduit sur un milieu gazeux comprenant des agents biologiques nocifs, toxiques, ou pathogènes. Le cas échéant, l'inactivation consiste le plus souvent à dénuer les agents de leur caractère nocif, toxique, ou pathogène, notamment en inhibant leur capacité de réplication (reproduction).

Le "milieu gazeux" au sein duquel sont dispersés les agents biologiques précités est en général de l'air, mais il peut éventuellement s'agir d'un autre gaz contaminé par des agents biologiques. Notamment pour obtenir une efficacité suffisante du procédé de décontamination, il est toutefois préférable que le milieu gazeux contienne de l'oxygène.

Selon un mode de réalisation particulier, le milieu gazeux comprenant les espèces biologiques à l'état dispersé se présente sous la forme d'un aérosol qui comprend de fines gouttelettes liquides, en général des gouttelettes d'eau, dispersées au sein du milieu gazeux, les agents biologiques étant présents, en tout ou partie, au sein de ces gouttelettes.

Ainsi, par exemple, le milieu gazeux traité selon le procédé de l'invention peut être un aérosol comprenant de l'air à titre de milieu gazeux dispersant et contenant des gouttelettes d'eau incluant des bactéries et/ou des virus, par exemple des bactéries de type du genre *Legionella,* comme la *Legionella pneumophilia.*

Selon un autre mode de réalisation, les espèces biologiques sont simplement dispersées en tant que telles au sein du milieu gazeux. Selon ce mode, le milieu gazeux traité peut par exemple être de l'air contenant des virus, des spores et/ou des champignons en suspension, ces espèces étant éventuellement déposées sur des supports particulaires tels que des poussières ou bien des particules de sable.

Quelle que soit la nature des agents biologiques et du milieu gazeux, le procédé d'inactivation de la présente invention est effectué en mettant en contact le milieu gazeux contenant les agents biologiques avec un matériau semi-conducteur photoactivé, ce matériau étant de préférence de l'oxyde de titane photoactivé.

Par "matériau semi-conducteur", on entend, au sens de la présente invention un matériau où les états électroniques ont un spectre de bande comprenant une bande de valence et une bande de conduction séparées par une bande interdite, et où l'énergie nécessaire pour faire passer un électron de ladite bande de valence à ladite bande de conduction est de préférence comprise entre 1,5 eV et 4 eV. A titre de tels matériaux semi-conducteurs, on peut notamment citer l'oxyde de titane, ou bien encore d'autres oxydes métalliques tels que WO₃ ZnO ou SnO₂ ou bien des sulfures métalliques tels que CdS, ZnS ou WS₂ ou encore d'autres composés tels que GaAs, GaP, CdSe ou SiC. Selon la présente invention, on utilise préférentiellement l'oxyde de titane qui conduit à des résultats particulièrement satisfaisants.

Au sens de la présente description, le terme de "matériau semi-conducteur photoactivé" désigne un matériau semi-conducteur du type précité qui a été soumis à un rayonnement comprenant des photons d'énergie supérieure ou égale à l'énergie nécessaire pour promouvoir les électrons de la bande de valence vers la bande de conduction (énergie dite "gap" entre les bandes de valence et de conduction).

Ainsi, au sens de la présente description, on entend en particulier par "oxyde de titane photoactivé" un oxyde de titane soumis à un rayonnement comprenant des photons d'énergie supérieure ou égale à l'énergie nécessaire pour promouvoir les électrons de la bande de valence vers la bande de conduction, typiquement un rayonnement contenant des photons d'énergie supérieure à 3 eV, de préférence à 3,2 eV, et en particulier un rayonnement comprenant des longueurs d'ondes inférieures ou égales à 400 nm, par exemple inférieures ou égales à 380 nm. A titre de tels rayonnements, on peut notamment citer les rayonnements fournis par les lampes à rayonnements ultraviolets du type des lampes dites "à lumière noire".

Il est connu que, dans un matériau semi-conducteur photoactivé, et en particulier dans un oxyde de titane photoactivé, il se crée, sous l'effet d'un rayonnement de type précité, des paires électrons/trous (un "trou" étant un déficit électronique dans la couche de valence laissé lors du "saut" d'un électron vers la bande de conduction), ce qui confère au matériau semi-conducteur photoactivé des propriétés d'oxydo-réduction prononcées. Ces propriétés d'oxydo-réduction sont particulièrement prononcées dans le cas de l'oxyde de titane photoactivé, qui sont mises à profit dans de nombreuses applications photocatalytiques de l'oxyde de titane.

Les inventeurs ont mis en évidence qu'un matériau semi-conducteur photoactivé tel qu'un oxyde de titane photoactivé s'avère en fait suffisamment actif pour permettre l'inactivation d'agents biologiques dans un milieu gazeux où lesdits agents biologiques sont pourtant fortement dispersés. Dans ce cadre, il est notamment surprenant de constater que le procédé de l'invention permet, par exemple, de traiter efficacement des milieux gazeux très dilués, à savoir comprenant moins de 10⁻³ agents biologiques par cm³, voire moins de 10⁻⁴ agents biologiques par cm³. L'activité d'un matériau semi-conducteur photoactivé de type oxyde de titane photoactivé se révèle par ailleurs suffisante pour traiter efficacement des milieux gazeux ayant des teneurs élevées en agents biologiques, par exemple des milieux gazeux contenant plus de 1 agent biologique par cm³, et même plus de 10 agents biologiques par cm³ dans la plupart des cas, et ce même avec des débits importants de flux gazeux par exemple de l'ordre de 1 à 10 litres par minute. Ainsi, le procédé de l'invention permet, en règle générale, de traiter efficacement des milieux gazeux contenant typiquement entre 10⁻⁴ et 10 agents biologiques par cm³, par exemple entre 5.10⁻³ et 5 agents biologiques par cm³, et notamment des milieux dilués contenant entre 10⁻⁴ et 0,1 agents biologiques par cm³ ou bien des milieux concentrés contenant entre 0,1 et 10 agents biologiques par cm³.

Sans vouloir être lié à une théorie particulière, les résultats des travaux des inventeurs permettent d'avancer que cette inactivation des agents biologiques est rendue possible par le caractère fortement oxydant du matériau semi-conducteur photoactivé qui semble induire une dégradation photocatalytique des agents biologiques qui est initiée lorsque ces agents viennent en contact avec la surface du semi-conducteur photoactivé, et qui se poursuit ensuite sans avoir à maintenir un contact entre le semi-conducteur et l'agent biologique à inactiver, ce qui rend possible le traitement efficace d'un milieu aussi dilué qu'une dispersion gazeuse telle qu'un aérosol. Ces phénomènes sont tout particulièrement prononcés dans le cas où le semi-conducteur utilisé est l'oxyde de titane.

Dans la présente invention, pour augmenter encore l'efficacité des mécanismes d'oxydation précités, il peut être avantageux d'utiliser, conjointement au matériau semi-conducteur photoactivé, d'autres matériaux présentant un caractère oxydant. Dans ce cadre, on peut en particulier utiliser des matériaux à base d'un semi-conducteur tel que l'oxyde de titane et contenant en outre des métaux tels que de l'or ou de l'argent sous forme métallique, par exemple sous forme de particules dispersées dans le matériau semi-conducteur ou bien déposées sur sa surface.

Selon ce mode de réalisation particulier, le rapport massique (oxydant additionnel/semi-conducteur) reste avantageusement inférieur à 5%, voire à 3%, et il est typiquement compris entre 0,5 et 2%. La présence de tels oxydants additionnels n'est toutefois pas requise, dans le cas général, pour obtenir un traitement efficace.

De plus, la dégradation photocatalytique d'agents biologiques qui a été observée par les inventeurs présente l'avantage d'être très peu sélective, ce qui signifie que tout agent biologique est en principe susceptible d'être dégradé par une mise en contact avec un semi-conducteur photoactivé de type oxyde de titane photoactivé. En pratique, il s'avère que l'oxyde de titane photoactivé présente effectivement un très large spectre de décontamination.

Un autre avantage du procédé est que la dégradation photocatalytique particulièrement efficace qui est observée avec un semi-conducteur photoactivé de type oxyde de titane photoactivé est obtenue très simplement et à coût réduit, dans la mesure où elle ne nécessite qu'une irradiation avec des rayonnements de relativement faible énergie. Ainsi, dans le cas de l'oxyde de titane par exemple, seules des énergies de rayonnement de l'ordre de 3 à 3,2 eV, c'est-à-dire de longueurs de l'ordre de 380 à 400 nm, sont requises. L'énergie requise pour la photoactivation peut encore être réduite si le matériau semi-conducteur est dopé (par exemple par des métaux tels que le chrome ou bien par des composés à base de N, S ou C) ou bien encore en utilisant des agents chromophores (anthracènes ou anthracines par exemple), en association avec le matériau semi-conducteur. Dans ce cas, des énergies d'activation très faibles peuvent être suffisantes pour photoactiver le matériau, qui peuvent par exemple correspondre à des longueurs d'ondes supérieures ou égales à 500 nm, par exemple supérieures ou égales à 550 nm.

L'irradiation de l'oxyde de titane est en général effectuée sous un rayonnement contenant des rayonnements de la gamme du proche ultraviolet, par exemple par irradiation par la lumière solaire ou bien encore par des lampes à vapeur de sodium ou bien par des lampes dite "à lumière noire", qui sont des rayonnements qu'on peut obtenir avec un faible coût. Par ailleurs, il est à noter que le rayonnement utilisé pour photoactiver le TiO₂, ou plus généralement le semi-conducteur est, en général, un rayonnement d'énergie insuffisante pour assurer à lui seul une inactivation des agents biologiques, en l'absence de semi-conducteur de type de TiO₂.

En d'autres termes, les rayonnements utilisés pour photoactiver le semi-conducteur dans le procédé de l'invention ne sont généralement pas, en soi, des rayonnements d'énergie suffisante pour assurer un effet germicide. Les rayonnements utilisés pour photoactiver les matériaux semi-conducteurs selon le procédé de l'invention ont ainsi, en général, des longueurs d'ondes supérieures à 254 nm, et typiquement supérieures à 320 nm, par exemple supérieures ou égales à 350 nm.

Il est en outre à souligner qu'aucun chauffage n'est requis pour réaliser la photoactivation de l'oxyde de titane, ce qui permet d'effectuer le procédé de l'invention à température ambiante, par exemple entre 10 et 30°C.

La nature exacte du matériau semi-conducteur utilisé selon l'invention n'est, en règle générale, pas déterminante pour obtenir l'effet recherché d'inactivation des agents biologiques.

Ainsi, dans le cas de l'oxyde de titane, par exemple, tout oxyde de titane commercial peut être utilisé efficacement dans le procédé de l'invention, ce qui constitue encore un avantage du procédé.

Néanmoins, selon un mode de réalisation conduisant à de bons résultats en termes d'inactivation des agents biologiques, l'oxyde de titane utilisé selon le procédé de l'invention contient du TiO₂ de forme anatase, de préférence à raison d'au moins 50%. Ainsi, selon ce mode de réalisation, l'oxyde de titane utilisé peut par exemple être constitué pour l'essentiel (à savoir en général pour au moins 99% en masse, et de préférence pour au moins 99,5% en masse, voire pour au moins 99,9% en masse) de TiO₂ de forme anatase.

L'utilisation de TiO₂ sous forme rutile se révèle également intéressante, dans la mesure où le TiO₂ sous cette forme est photoactivé par le spectre de la lumière visible.

Selon un autre mode de réalisation intéressant, l'oxyde de titane utilisé comprend un mélange de TiO₂ de forme anatase et de TiO₂ de forme rutile, avec une proportion d'anatase/rutile de préférence entre 50/50 et 99/1, par exemple entre 70/30 et 90/10, et typiquement de l'ordre de 80/20.

Par ailleurs, notamment pour optimiser les échanges entre le matériau semi-conducteur de type oxyde de titane et les agents biologiques dispersés dans le flux gazeux, il est le plus souvent avantageux que le matériau semi-conducteur utilisé ait une surface spécifique comprise entre 20 et 500 m²/g, de préférence supérieure ou égale à 40m²/g, et encore plus avantageusement au moins égale à 100 m²/g et ce, tout particulièrement lorsqu'il s'agit d'oxyde de titane. La surface spécifique à laquelle il est fait référence ici est la surface spécifique BET mesurée par adsorption d'azote selon la technique dite de Brunauer-Emmet-Teller. A cet effet, on peut notamment utiliser un oxyde de titane présentant en tant que tel une haute surface spécifique, ou bien un oxyde de titane déposé sur un support poreux (tel que, par exemple, un support de silice) présentant une haute surface spécifique.

A titre d'oxyde de titane particulièrement avantageux dans le procédé de l'invention, on pourra notamment utiliser l'oxyde de titane commercialisé par la société Degussa sous le nom de TiO₂ de type P25.

Le matériau semi-conducteur photoactivé qui est utilisé selon l'invention peut se présenter sous différentes formes physiques, en fonction du milieu gazeux traité, et notamment en fonction du volume de ce milieu et de la vitesse à laquelle on souhaite effectuer le traitement. De façon générale, le matériau semi-conducteur de type oxyde de titane peut être utilisé sous toute forme adaptée à son irradiation par un rayonnement de longueur d'onde permettant sa photoactivation et permettant la mise en contact de l'oxyde de titane à l'état photoactivé avec les agents biologiques à traiter, sous réserve qu'il soit accessible pour inactiver les agents biologiques.

Dans le procédé de l'invention, le matériau semi-conducteur de type oxyde de titane utilisé est mis en oeuvre à l'état immobilisé sur la surface interne du réacteur, le milieu gazeux à traiter étant mis en contact avec cette surface modifiée. Selon un mode particulier, la surface sur laquelle est immobilisé le matériau semi-conducteur de type oxyde de titane peut être une surface sur laquelle est déposé un support de haute surface spécifique (par exemple une couche de silice), le matériau semi-conducteur étant immobilisé sur ce support. Selon un autre mode de réalisation, le matériau semi-conducteur de type oxyde de titane peut être mis en oeuvre sous la forme d'un dépôt obtenu en déposant un film d'une dispersion (par exemple une dispersion aqueuse) de particules à base dudit semi-conducteur de type oxyde de titane, sur une surface et en séchant ensuite le film obtenu.

Selon un autre aspect particulier, la présente invention a également pour objet un dispositif adapté à la mise en oeuvre du procédé précité d'inactivation d'agents biologiques dispersées au sein d'un milieu gazeux.

Ce dispositif comporte un réacteur comprenant :
- une surface interne délimitant un passage s'étendant suivant une ligne, ladite surface interne comportant une pluralité de saillies disposées en hélice autour de ladite ligne ;
- une entrée adaptée à l'introduction du milieu gazeux contenant les agents biologiques dispersés dans le passage ; et
- une sortie adaptée à l'évacuation du milieu gazeux après inactivation des agents biologiques,

De plus, ledit réacteur comprend, à titre de moyen d'inactivation d'agents biologiques dispersés au sein d'un milieu gazeux, un dépôt d'un matériau semi-conducteur (de préférence un dépôt d'oxyde de titane) immobilisé sur lesdites saillies de sa surface interne, associé à des moyens d'irradiation dudit dépôt d'oxyde de titane capable de photoactiver l'oxyde de titane dudit dépôt en présence du milieu gazeux contenant les agents biologiques.

Le dépôt à base de matériau semi-conducteur qui est présent sur la surface interne du réacteur est de préférence un dépôt d'oxyde de titane choisi parmi les oxydes de titane préférentiels précités. Ainsi, il s'agit avantageusement d'un oxyde de titane contenant du TiO₂ de forme anatase ou un mélange rutile/anatase et présentant une surface spécifique comprise entre 20 et 500 m²/g. Ce dépôt peut par exemple être obtenu en déposant un film d'une dispersion (par exemple une dispersion aqueuse) de particules à base de matériau semi-conducteur de type oxyde de titane sur la surface interne du réacteur, puis en séchant le film obtenu. Ce dépôt peut également être obtenu par séchage d'un film d'une dispersion dans un solvant non aqueux ou le semi-conducteur utilisé n'est pas soluble. Dans le cas où le semi-conducteur photoactivé utilisé est de l'oxyde de titane, un dépôt d'oxyde de titane sur la surface peut être réalisé en déposant une solution de titanate et en traitant thermiquement le dépôt ainsi obtenu, ce par quoi on obtient la formation de TiO₂ à partir du précurseur titanate.

Le dépôt de matériau semi-conducteur de type oxyde de titane peut être de nature continue ou discontinue, et il s'agit de préférence d'un film solide continu réparti sur la totalité de la surface interne du réacteur, notamment pour optimiser la surface d'échange entre le flux gazeux et l'oxyde de titane photoactivé. Par ailleurs, ce dépôt a de préférence une épaisseur moyenne comprise entre 0,5 µm et 100 µm, par exemple entre 1 et 20 µm, cette épaisseur étant typiquement de l'ordre de 5 µm.

Les moyens d'irradiations associés à ce dépôt de semi-conducteur de type oxyde de titane sont en général des sources de rayonnement comprenant des photons d'énergie supérieure à 3 eV (de préférence supérieure à 3,2 eV), par exemple une ou plusieurs lampes émettant des rayonnements comprenant des longueurs d'ondes inférieures à 300 nm (par exemple inférieures à 400nm), par exemple des lampes de type lampes à lumière noire ou à lumière visible. Selon un mode de réalisation particulier, la source de rayonnement utilisée peut être la lumière solaire. En général, ces sources de rayonnement sont localisées à l'extérieur du réacteur. Le cas échéant, pour permettre une activation de l'oxyde de titane, la paroi du réacteur est constituée d'un matériau transparent à au moins une partie du rayonnement efficace émis par les sources, c'est-à-dire que la paroi du réacteur laisse passer au moins une partie des rayonnements qui ont une énergie suffisante pour activer l'oxyde de titane. A cet effet, on préfère en général utiliser des réacteurs en verre, notamment en pyrex.

Le dispositif de l'invention permet d'inactiver de façon efficace les agents biologiques présents dans le flux gazeux. Le réacteur utilisé présente en outre l'avantage de pouvoir être utilisé dans toutes les positions (à savoir horizontale, verticale ou inclinée), notamment compte tenu de l'immobilisation de l'oxyde de titane sur les parois du réacteur. De plus, son entrée et sa sortie peuvent être permutées, ce qui permet, si nécessaire, d'inverser le sens du flux gazeux traité.

Notamment en fonction de la quantité de milieu gazeux à traiter et du débit envisagé, différentes dimensions et géométries peuvent être envisagées pour le réacteur.

Les saillies présentes sur la surface interne du réacteur sont avantageusement venues de matière avec la paroi du réacteur. De telles saillies sont particulièrement aisées à obtenir dans le cas où on utilise un réacteur en pyrex : dans ce cadre, les saillies peuvent être réalisées en déformant à chaud la paroi du réacteur.

Le nombre et la géométrie des saillies peuvent varier en une assez large mesure, notamment en fonction de l'application recherchée et du débit du flux gazeux traité. Il va de soi que, pour une géométrie de saillie donnée, la probabilité de rencontre entre les agents biologiques et l'oxyde de titane photoactivé augmente avec le nombre de saillies. Cela étant, dans le même temps, la présence de saillies est susceptible d'induire des pertes de charge dans le réacteur, qui sont d'autant plus importantes que le nombre de saillies augmente, en particulier dans le cas de réacteurs de dimensions importantes.

Pour limiter les pertes de charge de ce type tout en maintenant des probabilités élevées de contacts entre les agents biologiques du flux gazeux et l'oxyde de titane photoactivé, outre la disposition des saillies en hélice, on peut également jouer sur la géométrie des saillies et sur leur disposition sur la surface interne du réacteur. Dans ce cadre, il est à noter que les saillies peuvent présenter des surfaces orientées à co-courant ou à contre-courant. Si on souhaite limiter les pertes de charges, il est préférable de choisir des saillies présentant des surfaces orientées à co-courant. Dans ce cadre, les saillies peuvent avantageusement présenter une forme conique, ce qui permet, si nécessaire, d'inverser le sens du flux gazeux dans le réacteur tout en maintenant une orientation des saillies à co-courant.

Le procédé et le dispositif de l'invention vont maintenant être décrits plus en détails dans la description suivante, faite en référence aux dessins ci-annexés, dans lesquels :
- la Figure 1 représente une vue schématique de côté en élévation d'un dispositif selon un mode particulièrement intéressant de l'invention ; et
- la Figure 2 représente une vue schématique en coupe du réacteur de la Figure 1.

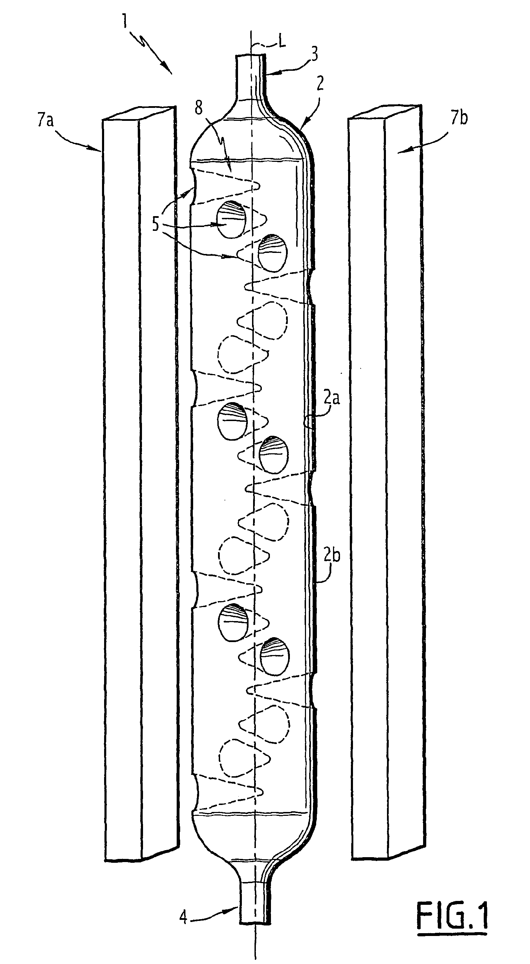

Le dispositif 1 des Figures ci-annexées comporte un réacteur tubulaire 2 constitué d'un tube en pyrex resserré à ses deux extrémités pour former une entrée 3 et une sortie 4 respectivement reliées à des moyens de conduction du flux gazeux à traiter (non représentés). Le réacteur 2 présente une surface interne 2a définissant dans le réacteur 2 un passage s'étendant suivant une ligne L entre l'entrée 3 et la sortie 4. Le réacteur 2 comporte une série de saillies 5, avantageusement (mais non nécessairement) sensiblement coniques, venues de matière avec la surface interne 2a du réacteur 2 et s'étendant radialement vers l'intérieur du réacteur 2. Ces saillies 5 peuvent par exemple être obtenues en déformant à chaud le tube de pyrex à l'aide d'un poinçon appliqué sur la surface externe 2b du réacteur. Les saillies 5 se retrouvent alors en creux sur la surface extérieure 2b. Les saillies 5 sont disposées selon une ligne hélicoïdale sur la surface interne du réacteur, comme visible sur la Figure 1. En d'autres termes, les saillies sont disposées en hélice autour de la ligne L.

Le réacteur 2 comprend, sur sa surface interne, un dépôt de matériau semi-conducteur, de préférence un dépôt d'oxyde de titane 6 (non représenté sur la Figure 1), qui peut être obtenu en déposant un film d'une dispersion aqueuse de particules de semi-conducteur de type oxyde de titane sur la surface interne du réacteur, puis en séchant le film obtenu et en répétant éventuellement ces opérations. Le film a typiquement une épaisseur de l'ordre de 5 µm (microns), cette épaisseur pouvant être modulée notamment en jouant sur la concentration initiale de la dispersion de particules d'oxyde de titane et sur le nombre de cycles de dépôt/séchage. Le dépôt 6 recouvre en particulier les saillies 5.

Outre le réacteur 2, le dispositif 1 comprend en outre des lampes 7a et 7b émettant des rayonnements comprenant, entre autres, des longueurs d'ondes inférieures ou égales à 400 nm et de préférence inférieures ou égales à 380 nm (typiquement les lampes 7a et 7b peuvent être des lampes à émission UV (de type "lumière noire") ou bien des lampes à émission de lumière visible, disposées de façon à irradier l'ensemble du dépôt d'oxyde de titane 6. Sur les figures, seules deux lampes sont représentées, mais en pratique, le nombre de lampes peut varier ainsi que leur puissance. A titre indicatif, pour un réacteur cylindrique de 6 cm de diamètre et de 30 cm de long, 4 lampes d'une puissance de 8 W de type tubes à lumière noire (par exemple du type de ceux commercialisés sous le nom de TL8W-08 par la société Philips), réparties autour du réacteur sont en général suffisantes.

Lors de la mise en oeuvre du dispositif 1 pour réaliser le procédé d'inactivation de l'invention, un flux gazeux comprenant des agents biologiques en suspension est introduit dans le réacteur par l'entrée 3. Le dispositif comprend en général à cet effet, en amont de l'entrée 3, des moyens d'injection non représentés sur les figures. En plus de ces moyens d'injection, il peut être intéressant que le dispositif comporte des moyens de dérivation d'une partie du flux gazeux entrant, associés à des moyens d'analyse qualitative et/ou quantitative des agents biologiques dans le flux gazeux, ces moyens d'analyse comprenant par exemple des membranes permettant la collecte des agents biologiques.

Le flux gazeux introduit dans le réacteur entre alors en contact avec le dépôt d'oxyde 6 présent sur la paroi interne du réacteur, qui est photoactivé sous l'effet du rayonnement fourni par les lampes 7a et 7b.

Les contacts entre les agents biologiques et l'oxyde de titane photoactivé ont lieu pour l'essentiel au niveau des surfaces supérieures 8 des saillies 5. Compte tenu de la forme sensiblement conique de ces saillies, les surfaces supérieures 8 des saillies sont orientés à co-courant et limitent ainsi les pertes de charge. II est à noter que le réacteur présente une symétrie parfaite et qu'il peut être utilisé en inversant le sens du courant (c'est-à-dire en intervertissant l'entrée et la sortie) en conservant les mêmes avantages.

La disposition des saillies 5 en hélice est également à même de limiter les pertes de charge au sein du réacteur, et ce tout particulièrement pour des réacteurs de dimensions importantes. Cette organisation particulière des saillies induit en outre un mouvement de convection du flux gazeux qui induit en outre un brassage qui permet une mise en contact des différents agents biologiques présents en suspension avec le dépôt 6 photoactivé.

Compte tenu de ces conditions, le dispositif 1 peut être utilisé avec des débits gazeux typiquement compris entre 1 et 10 litres par minute, et ce avec une bonne efficacité du processus d'inactivation des agents biologiques.

Ainsi, à la sortie 4 du réacteur, on récupère en général un flux gazeux où la majeure partie des agents biologiques sont inactivés.

Le dispositif 1 peut comprendre, en aval de la sortie 4, des moyens de dérivation d'une partie du flux gazeux sortant, associés à des moyens d'analyse qualitative et/ou quantitative des agents biologiques dans le flux gazeux, analogues à ceux qui peuvent être présents en amont de l'entrée 3.

Le procédé de l'invention, en particulier lorsqu'il est mis en oeuvre au moyen d'un dispositif tel que décrit en détails ci-dessus se révèle particulièrement efficace pour la décontamination de milieu gazeux contenant des agents biologiques nocifs, toxiques ou pathogènes.

Cette utilisation constitue, selon un autre aspect, un autre objet de la présente invention.

Différentes caractéristiques et avantages de l'invention ressortiront encore de l'exemple illustratif exposé ci-après.

### EXEMPLE

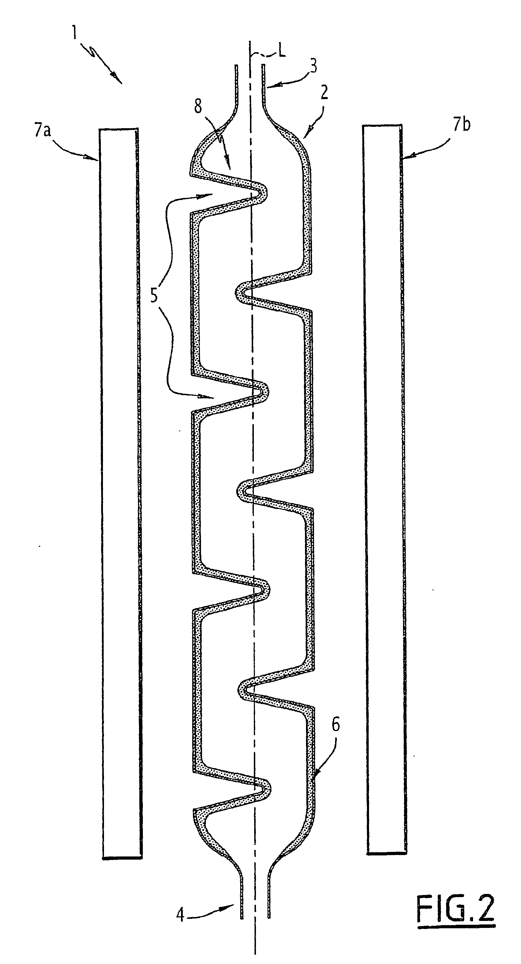

On a réalisé le traitement de différents aérosols comprenant des bactéries *Escherichia Coli* au moyen d'un dispositif tel que décrit sur les figures 1 et 2 ci-annexées et présentant les caractéristiques suivantes :
- diamètre du réacteur : 6 cm
- longueur du réacteur : 30 cm
- oxyde de titane utilisé : TiO₂ de type P25, commercialisé par la société Degussa, (oxyde de titane ayant un rapport des formes rutile/anatase de 20/80) présent dans le réacteur sous forme d'un dépôt d'épaisseur moyenne d'environ 5 µm et de BET égale à 50 m²/g.
- lampes utilisées : 4 lampes à rayonnement UV (tube de lumière noire) de type TL8W-08 commercialisées par la société Philips, ayant chacune une puissance de 8 W, orientées vers le réacteur et disposées autour du réacteur à une distance de 3 cm des parois.

Deux types d'aérosols ont été formés à partir de deux suspensions aqueuses d'*Escherichia Coli* comprenant respectivement 10⁷ et 10⁸ UFC par litre. (UFC = Unités Formant des Colonies). Ces aérosols ont été produits en injectant de l'air comprimé sous une pression de 1,5 bar (1,5. 10⁻⁵ Pa) dans chacune des suspensions aqueuses de bactéries *Escherichia coli,* à l'aide d'une buse de type Laskin, en utilisant un générateur d'aérosol de type PLG 2000 commercialisé par la société PALAS.

Dans chaque cas, on a quantifié la teneur en bactéries formant des colonies dans l'aérosol à l'entrée et à la sortie du réacteur. Cette quantification a été réalisée en incubant sur une gélose nutritive les bactéries filtrées obtenues respectivement en filtrant le flux entrant et le flux sortant du réacteur en régime permanent pendant une durée de 5 minutes sur un filtre membranaire en ester de cellulose ayant un diamètre de pore de 0,45 µm (Millipore).

Les conditions des essais réalisés et les résultats obtenus sont reportés dans les tableaux I est II ci-après.

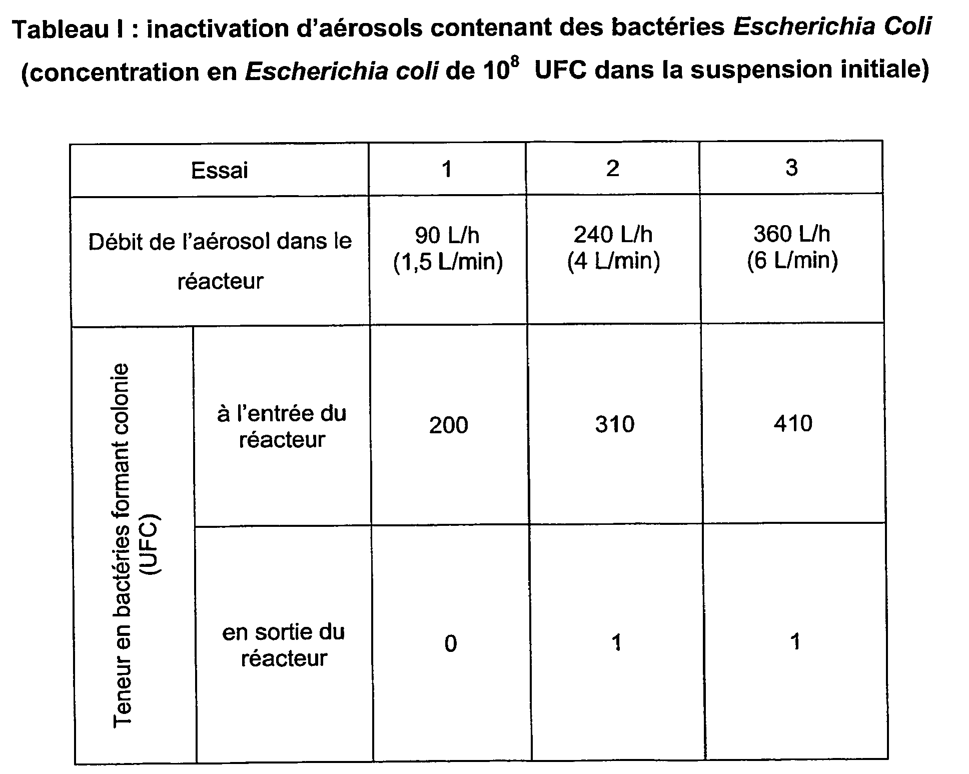

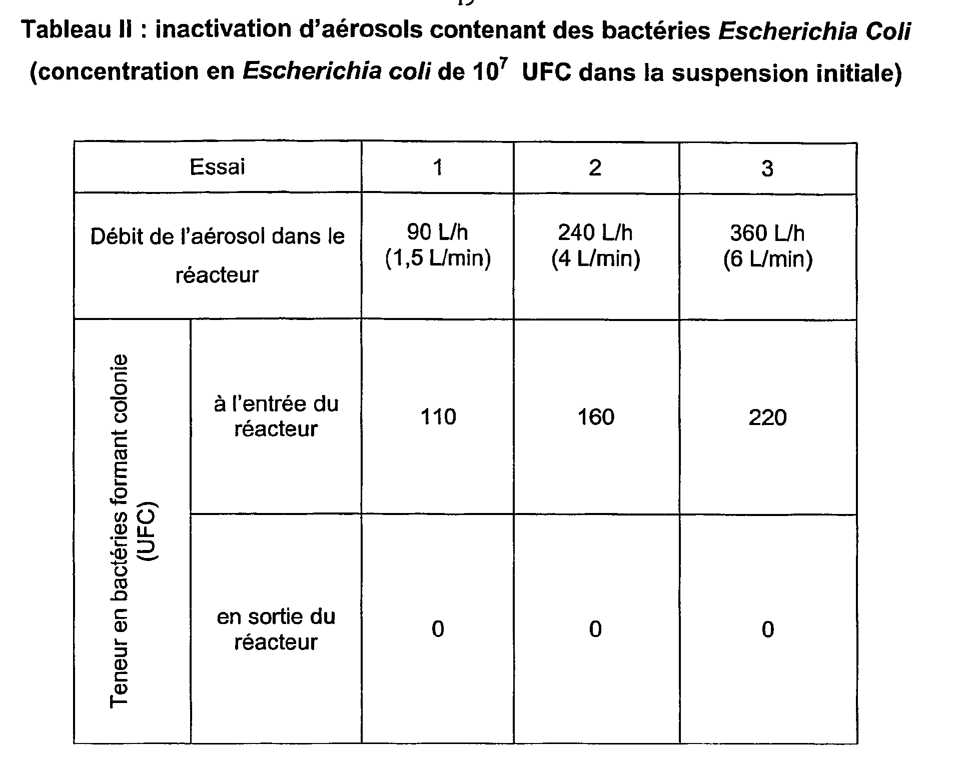

On a par ailleurs observé dans chaque cas les bactéries filtrées à l'entrée et à la sortie du réacteur par microscopie à épifluorescence utilisant le chlorure de 5-cyano-2,3-ditolylterazolium, de façon a identifier les bactéries qui respirent (qui apparaissent en rouge) et les autres. Dans ce cas, les filtres qui ont été utilisés sont des filtres membranaires en polycarbonate. Dans tous les cas, on observe à l'entrée du réacteur une population de bactéries dont l'essentiel apparaît avec une coloration rouge caractéristique des phénomènes de respiration. A l'inverse, à la sortie du réacteur, la majeure partie des bactéries observées ne présente plus cette coloration rouge.

## Revendications

1. Procédé d'inactivation d'agents biologiques dispersés au sein d'un milieu gazeux, qui comprend une étape consistant à mettre ledit milieu gazeux en contact avec un matériau semi-conducteur photoactivé au sein d'un réacteur (2) dont la surface interne comporte une pluralité de saillies (5) disposées en hélice autour d'une ligne (L), ledit matériau semi-conducteur photoactivé étant mis en oeuvre sous la forme d'un dépôt (6) sur la surface interne dudit réacteur, au moins sur lesdites saillies (5).

2. Procédé selon la revendication 1, où le matériau semi-conducteur photoactivé utilisé est de l'oxyde de titane photoactivé.

3. Procédé selon la revendication 1 ou la revendication 2, ledit procédé étant conduit dans un dispositif (1) qui comporte :
- ledit réacteur (2), ce réacteur comprenant une surface interne délimitant un passage s'étendant suivant une ligne (L), ladite surface interne comportant une pluralité de saillies (5) disposées en hélice autour de la ligne (L), une entrée (3) adaptée à l'introduction du milieu gazeux contenant les agents biologiques dispersés dans le passage, et une sortie (4) adaptée à l'évacuation du milieu après traitement, ledit réacteur comprenant ledit dépôt de matériau semi-conducteur (6) déposé sur les saillies (5) de sa surface interne, et
- des moyens d'irradiation (7a, 7b) dudit dépôt de matériau semi-conducteur, qui photoactivent le matériau semi-conducteur du dépôt (6) en présence du milieu gazeux contenant les agents biologiques.

4. Procédé selon l'une quelconque des revendications 1 à 3, où les agents biologiques dispersés dans le milieu gazeux sont des bactéries.

5. Procédé selon l'une des revendications 1 à 4, où le milieu gazeux comprenant les espèces biologiques à l'état dispersé se présente sous la forme d'un aérosol comprenant de fines gouttelettes liquides dispersées au sein du milieu gazeux, les agents biologiques étant présents, en tout ou partie, au sein de ces gouttelettes.

6. Procédé selon la revendication 5, où le milieu gazeux est un aérosol comprenant de l'air à titre de milieu gazeux dispersant et contenant des gouttelettes d'eau incluant des bactéries et/ou des virus.

7. Procédé selon la revendication 6, où les gouttelettes incluent des bactéries du genre *Legionella,* comme *Legionella pneumophilia.*

8. Procédé selon l'une quelconque des revendications 1 à 7, où les agents biologiques sont présents dans le milieux gazeux à une teneur comprise entre 10⁻⁴ et 10 agents biologiques par cm³.

9. Procédé selon la revendication 8, où l'oxyde de titane photoactivé utilisé est un oxyde de titane soumis à un rayonnement comprenant des longueurs d'onde comprises entre 254 et 400 nm.

10. Procédé selon l'une quelconque des revendications 1 à 9, où le matériau semi-conducteur utilisé est un oxyde de titane contenant du TiO₂ de forme anatase.

11. Procédé selon la revendication 10, où l'oxyde de titane utilisé comprend un mélange de TiO₂ de forme anatase et de TiO₂ de forme rutile, avec une proportion d'anatase/rutile entre 50/50 et 99/1, par exemple entre 70/30 et 90/10.

12. Procédé selon l'une des revendications 1 à 11, où le matériau semi-conducteur utilisé a une surface spécifique comprise entre 20 et 500 m²/g.

13. Dispositif (1) pour la mise en oeuvre d'un procédé d'inactivation d'agents biologiques dispersées au sein d'un milieu gazeux selon l'une quelconque des revendications 1 à 12, comportant
- un réacteur (2) comprenant une surface interne délimitant un passage s'étendant suivant une ligne (L), ladite surface interne comportant une pluralité de saillies (5) disposées en hélice autour de la ligne (L), une entrée (3) adaptée à l'introduction du milieu gazeux contenant les agents biologiques dispersés dans le passage, et une sortie (4) adaptée à l'évacuation du milieu après traitement, ledit réacteur comprenant, à titre de moyen d'inactivation d'agents biologiques dispersés au sein d'un milieu gazeux, un dépôt de matériau semi-conducteur (6) déposé sur lesdites saillies (5) de sa surface interne, et
- des moyens d'irradiation (7a, 7b) dudit dépôt de matériau semi-conducteur, capables de photoactiver le matériau semi-conducteur dudit dépôt (6) en présence du milieu gazeux contenant les agents biologiques.

14. Dispositif (1) selon la revendication 13, où le dépôt de matériau semi-conducteur (6) est un dépôt d'oxyde de titane.

15. Utilisation du procédé de l'une quelconque des revendications 1 à 12, pour la décontamination d'un milieu gazeux contenant des agents biologiques nocifs, toxiques ou pathogènes.

## Claims

1. Method for inactivating biological agents dispersed within a gaseous medium, which method comprises a step in which said gaseous medium is brought into contact with a photoactivated semiconductor material within a reactor (2) whose inside surface has a plurality of projections (5) which are arranged helically around a line (L), said photoactivated semiconductor material being used in the form of a deposit (6) on the inside surface of said reactor, at least on said projections (5).

2. Method according to claim 1, wherein the photoactivated semiconductor material used is photoactivated titanium oxide.

3. Method according to claim 1 or claim 2, said method being carried out in a device (1) which comprises:
- said reactor (2), the reactor having an inside surface which delimits a passage extending along a line (L), said inside surface having a plurality of projections (5) which are arranged helically around the line (L), an inlet (3) suitable for introducing the gaseous material containing the dispersed biological agents into the passage, and an outlet (4) suitable for discharging the medium after treatment, said reactor comprising said deposit of semiconductor material (6) deposited on the projections (5) of its inside surface, and
- means (7a, 7b) for irradiating said deposit of semiconductor material, which means photoactivate the semiconductor material of the deposit (6) in the presence of the gaseous medium containing the biological agents.

4. Method according to any one of claims 1 to 3, wherein the biological agents dispersed in the gaseous medium are bacteria.

5. Method according to any one of claims 1 to 4, wherein the gaseous medium containing the biological species in the dispersed state is in the form of an aerosol containing fine liquid droplets dispersed within the gaseous medium, the biological agents being present, wholly or partially, within the droplets.

6. Method according to claim 5, wherein the gaseous medium is an aerosol comprising air as the gaseous dispersing medium and containing water droplets including bacteria and/or viruses.

7. Method according to claim 6, wherein the droplets include bacteria of the genus *Legionella,* such as *Legionella pneumophilia.*

8. Method according to any one of claims 1 to 7, wherein the biological agents are present in the gaseous medium in an amount of from 10⁻⁴ to 10 biological agents per cm³.

9. Method according to claim 8, wherein the photoactivated titanium oxide used is a titanium oxide subjected to radiation comprising wavelengths of from 254 to 400 nm.

10. Method according to any one of claims 1 to 9, wherein the semiconductor material used is a titanium oxide containing TiO₂ of anatase form.

11. Method according to claim 10, wherein the titanium oxide used comprises a mixture of TiO₂ of anatase form and TiO₂ of rutile form, with an anatase/rutile ratio of from 50/50 to 99/1, for example from 70/30 to 90/10.

12. Method according to any one of claims 1 to 11, wherein the semiconductor material used has a specific surface area of from 20 to 500 m²/g.

13. Device (1) for carrying out a method for inactivating biological agents dispersed within a gaseous medium according to any one of claims 1 to 12, which device comprises
- a reactor (2) having an inside surface which delimits a passage extending along a line (L), said inside surface having a plurality of projections (5) which are arranged helically around the line (L), an inlet (3) suitable for introducing the gaseous medium containing the dispersed biological agents into the passage, and an outlet (4) suitable for discharging the medium after treatment, said reactor comprising, as means for inactivating biological agents dispersed within a gaseous medium, a deposit of semiconductor material (6) deposited on said projections (5) of its inside surface, and
- means (7a, 7b) for irradiating said deposit of semiconductor material, which means are capable of photoactivating the semiconductor material of said deposit (6) in the presence of the gaseous medium containing the biological agents.

14. Device (1) according to claim 13, wherein the deposit of semiconductor material (6) is a deposit of titanium oxide.

15. Use of the method of any one of claims 1 to 12 in the decontamination of a gaseous medium containing harmful, toxic or pathogenic biological agents.

## Patentansprüche

1. Verfahren zur Inaktivierung von in einem gasförmigen Medium verteilten biologischen Agentien, welches Verfahren einen Schritt umfasst, der darin besteht, das gasförmige Medium mit einem lichtaktivierten Halbleitermaterial in einem Reaktor (2) in Kontakt zu bringen, dessen innere Oberfläche eine Vielzahl von Vorsprüngen (5) aufweist, die schraubenförmig um eine Linie (L) angeordnet sind, wobei das lichtaktivierte Halbleitermaterial in Form einer Ablagerung (6) auf der inneren Oberfläche des Reaktors, zumindest auf den Vorsprüngen (5) ausgeführt ist.

2. Verfahren nach Anspruch 1, wobei das verwendete lichtaktivierte Halbleitermaterial lichtaktiviertes Titanoxid ist.

3. Verfahren nach Anspruch 1 oder Anspruch 2, wobei das Verfahren in einer Vorrichtung (1) durchgeführt wird, die umfasst:
- den Reaktor (2), wobei der Reaktor eine innere Oberfläche, die einen sich entlang einer Linie (L) erstreckenden Durchgang begrenzt, wobei die innere Oberfläche eine Vielzahl von Vorsprüngen (5) hat, die schraubenförmig um die Linie (L) angeordnet sind, einen Einlass (3), der zum Einleiten des gasförmigen, die biologischen Agentien enthaltenden Mediums in den Durchgang ausgelegt ist, und einen Auslass (4) aufweist, der zum Ausleiten des Mediums nach einer Behandlung ausgelegt ist, wobei der Reaktor die Ablagerung aus Halbleitermaterial (6) aufweist, das auf den Vorsprüngen (5) seiner inneren Oberfläche aufgebracht ist, und
- Bestrahlungseinrichtungen (7a, 7b) für die Ablagerung aus Halbleitermaterial, die das Halbleitermaterial der Ablagerung (6) im Beisein des gasförmigen, die biologischen Agentien enthaltenden Mediums mit Licht aktivieren.

4. Verfahren nach einem der Ansprüche 1 bis 3, wobei die in dem gasförmigen Medium verteilten biologischen Agentien Bakterien sind.

5. Verfahren nach einem der Ansprüche 1 bis 4, wobei das gasförmige Medium, das die biologischen Spezien im verteilten Zustand enthält, sich in Form eines Aerosols darstellt, das feine Flüssigkeitströpfchen enthält, die im gasförmigen Medium verteilt sind, wobei die biologischen Agentien ganz oder teilweise im Inneren dieser Tröpfchen vorhanden sind.

6. Verfahren nach Anspruch 5, wobei das gasförmige Medium ein Aerosol ist, das Luft als gasförmiges Medium enthält, das Wassertröpfchen verteilt und enthält, die Bakterien und/oder Viren beinhalten.

7. Verfahren nach Anspruch 6, wobei die Tröpfchen Bakterien der Art *Legionella,* wie *Legionella pneumophilia* beinhalten.

8. Verfahren nach einem der Ansprüche 1 bis 7, wobei die biologischen Agentien im gasförmigen Medium mit einem Anteil von zwischen 10⁻⁴ und 10 biologischen Agentien pro cm³ vorhanden sind.

9. Verfahren nach Anspruch 8, wobei das verwendete lichtaktivierte Titanoxid ein Titanoxid ist, das einer Strahlung ausgesetzt wird, die Wellenlängen umfasst, die zwischen 254 und 400 nm liegen.

10. Verfahren nach einem der Ansprüche 1 bis 9, wobei das verwendete Halbleitermaterial ein Titanoxid ist, das TiO₂ in Form von Anatas enthält.

11. Verfahren nach Anspruch 10, wobei das verwendete Titanoxid eine Mischung aus TiO₂ in Form von Anatas und TiO₂ in Form von Rutil umfasst, mit einem Verhältnis von Anatas/Rutil zwischen 50/50 und 99/1, zum Beispiel zwischen 70/30 und 90/10.

12. Verfahren nach einem der Ansprüche 1 bis 11, wobei das verwendete Halbleitermaterial eine spezifische Oberfläche aufweist, die zwischen 20 und 500 m²/g beträgt.

13. Vorrichtung (1) zur Durchführung eines Verfahrens nach einem der Ansprüche 1 bis 12 zur Inaktivierung von in einem gasförmigen Medium verteilten biologischen Agentien, die aufweist
- einen Reaktor (2) mit einer inneren Oberfläche, die einen sich entlang einer Linie (L) erstreckenden Durchgang begrenzt, wobei die innere Oberfläche eine Vielzahl von Vorsprüngen (5) aufweist, die schraubenförmig um die Linie (L) angeordnet sind, einem Einlass (3), der zum Einleiten des gasförmigen, die biologischen Agentien enthaltenden Mediums in den Durchgang ausgelegt ist, und einem Auslass (4), der zum Ausleiten des Mediums nach einer Behandlung ausgelegt ist, wobei das Reaktionsgefäß als Einrichtung zur Inaktivierung von im gasförmigen Medium verteilten biologischen Agentien eine Ablagerung aus Halbleitermaterial (6) aufweist, das auf den Vorsprüngen (5) seiner inneren Oberfläche aufgebracht ist, und
- Bestrahlungseinrichtungen (7a, 7b) für die Ablagerung aus Halbleitermaterial, die in der Lage sind, das Halbleitermaterial der Ablagerung (6) im Beisein des gasförmigen, die biologischen Agentien enthaltenden Mediums mit Licht zu aktivieren.

14. Vorrichtung (1) nach Anspruch 13, wobei die Ablagerung (6) aus Halbleitermaterial eine Titanoxidablage ist.

15. Verwendung des Verfahrens nach einem der Ansprüche 1 bis 12 zur Dekontaminierung eines gasförmigen Mediums, das schädliche, toxische oder pathogene biologische Agentien enthält.
